# EUROPEAN PATENT APPLICATION

(11) **EP 4 541 278 A2**
(43) Date of publication of application: **23.04.2025**
(21) Application number: 25163053.9
(22) Date of filing: 18.08.2021
(51) Int. Cl.: A61B 5/155

(54) **FINGER RECEPTACLE AND HOLDING DEVICE**

(30) Priority: 26.08.2020 GB 202013379
(62) Divisional of application: 21762769.4
(71) Applicant: Mowgli Innovations Ltd, London W9 3BB (GB)
(72) Inventor: HAINES, Arnaud, London, W9 3BB (GB); HAINES, Georges, London, W9 3BB (GB); ZOCHER, Sebastian, 01277 Dresden (DE)
(74) Representative: Stratagem IPM Limited

(57) **Abstract**

The invention concerns a finger holding device comprising a base, an elongate docking structure comprising a docking arm and docking foot; a finger receptacle for finger compression, wherein the docking arm is engaged with the finger receptacle and movable there along, such that the device has a closed configuration in which the receptacle is aligned alongside the docking arm and rests adjacent the docking foot and an open configuration in which the receptacle extends from the docking arm and is non-adjacent the docking foot; and an attachment interface associated with the base for releasably mounting an interchangeable collector attachment to the device. The invention further relates to devices for blood capture and/or blood collection that incorporate said finger holding device of the invention including a finger receptacle.

## Description

### Introduction

The invention relates to a novel finger receptacle, as well as an associated holding device. The invention further concerns kit assemblies comprising the receptacle and/or device for use in home-based blood/plasma capture and collection. The present invention and embodiments thereof permits a sufficiently large sample volume of blood and/or plasma for diagnostic and medical testing/analytical purposes to be collected by the user without a clinician.

### Background of the invention

Collection or capture of blood is routinely required for medical diagnostic testing to quickly and accurately identify conditions, disease, deficiencies and many other health issues in humans and animals.

As it concerns humans, members of the general population and those who have symptoms of an illness will require analysis of their blood or other key fluids derived therefrom thereto, such as plasma, at multiple times in their lives whether it be for specific diagnostic purposes or for regular health monitoring, for example, when a key biological change occurs such as development of an allergy, during pregnancy or other symptoms of an illness.

Further, where frequent blood collection is required during long term illness and/or for delicate or frail persons such as children, or the elderly, minimising trauma and the challenges of accessing veins in some of these patients during that process would be particularly desirable (for patients and clinicians alike).

Existing cutting methods to release blood for sample collection include skin incision devices according to, for example US 5,797,940 or US 6,221,089. Those with a cutting or slicing function creating a larger incision may enable up to around 400µl to be extracted from the incision. Often these devices are used by clinicians for patients already in hospital, or at a surgery where it is desirable to avoid a needle, for example with babies or young children. However, such devices do not provide a home user solution, as regards capture and collection of blood.

Where a sample, particularly a larger volume sample of 1ml is needed from an adult, a conventional needle and syringe is most effective and can be used to serially fill a plurality of vials to reach the total volume required for the number of tests ordered by the doctor. For this reason, those who are not in a hospital environment already, must visit either a specialist surgery or hospital clinic, where the blood can be collected by a trained phlebotomist and sent off to the lab thereafter. Such visits are inconvenient and burdensome to patients and the method is not particularly comfortable. The service is also costly to the health provider because a trained clinician is essential to undertake the procedure. Reducing the need for clinician-led multi-syringe/needle systems, especially for conventional blood testing would be beneficial for the health provider or service.

Some devices and methods for collection of blood are available at home. However, many of these involve a finger (or heel) prick test which releases around 100µL of blood and require repeat squeezing at the wound/puncture site causing blood haemolysis and coagulation, as well as causing blood spillage with associated risks. This volume can be sufficient for some very limited testing but in the majority of cases it is too little to be suitable for reliable and/or repeatable diagnostic testing. Obtaining a sample that could be sent to a laboratory directly but which is in a sufficient volume and of the required quality to process multiple analytical tests would therefore be useful.

Other home devices additionally offering a collection solution may include an inherent lancet integrated in the housing of the device, such as PCT2018/039305. Means to achieve a pricking (to effect blood release) and collection functions are proximal and housed in the same part of the device. A single pricking/lancing action first punctures the most distal surface tip of a finger followed by a manual pressurising or pumping action, around the finger, to subsequently force blood through the temporary aperture. However, such devices are not practically intended to collect blood volumes beyond around 500µl, which is around half the volume required to fulfil the technical problem described herein and sought by the present inventors. Such a device and methods neither improves flow rate nor facilitates easy extraction from the cutting site and so collecting larger amounts of blood from the single puncture site, in a painless way, is not achievable. Attempting to do so would cause significant discomfort to the user and thus would not meet the patient compliance requirements essential for a self-operated device of this type. Moreover, such devices do not afford flexibility with the cutting means/type and location of cutting, which is crucial to solving the problems described herein. These approaches can be time-consuming and messy and require the individual's full attention throughout the collection process.

There remains a need for a relatively pain-free, self-operated solution which facilitates improved blood capture; including the flow, release and collection of blood from a finger and a solution which helps facilitate sufficiently large volume samples of the required quality for multi-purpose diagnostic testing.

The invention comes about from a desire to meet these objectives.

### Summary of the invention

The invention concerns a finger receptacle comprising an elongated, flexible sleeve having a length defined between a proximal end and a distal end, the sleeve further comprising a support base, a ceiling and top surface defining a longitudinal axis X, whereby the sleeve is adapted to bias toward a radially contracted state and when urged into a radially expanded state effects a radial compression action about the length to releasably secure a finger within the receptacle; and at least one angled projection extending from the ceiling and top surface at the distal end of the sleeve, the at least one projection angled inwardly from the longitudinal axis X and adapted to contact a nail of the finger releasably secured in the sleeve at the distal end of the receptacle and effecting a second compression action.

When positioned fully within the receptacle a first compression, a radial compression, is exerted along the length of the finger, with the exception of the fingertip. The fingertip is pushed over the distal end and upon connection with the at least one angled projection is forced downwardly at an angle to overhang the edge of the support base. The receptacle of the invention therefore provides two different forces in different places; around the finger and on the nail. In some examples, the compression/force on the nail may be more evenly distributed for greater comfort; for example, in those embodiments in which the receptacle has two spaced projections contacting the nail, a more even pressure is applied thereacross (and downwardly at angle A) and a less severe force will be experienced at a single point on the nail.

Advantageously, such a receptacle is suited for use in applications where blood flow in the finger is required to be optimised for subsequent blood capture. This is achieved by providing structural features which result in a combination of compression actions. The radial compression acts to clamp or squeeze the body of the finger and thus increases blood flow to the tip of the finger. Blood flow can normally be enhanced by massaging externally with the other hand to further direct the blood flow to the distal part of the finger. However, in the present case an additional massaging action is possible but not essential. The second compression action provides a distal linear action in a generally downward direction, pushing directly on the nail. This second action enhances the localised blood flow at the tip of the finger towards the cutting zones and causes these areas to be locally engorged with blood increasing the blood flow rate and the amount released upon cutting. The two actions therefore provide a sufficient amount of high quality blood, avoiding the need for vigorous massage or pumping at a wound site, which sometimes causes damage to the cells and reduces sample quality. The two compressive actions in combination therefore enhance the technical function of the receptacle.

Additionally, the surrounding compression of the sleeve smooths the surface of the finger along its length, providing a more consistent and even pressure than many of the examples in the art, thereby further contributing to the technical advantages that result from use, described herein.

In embodiments, the base and the ceiling of the receptacle are flexibly connected. In examples, the flexible connection is made possible by an expandable and/or memory section or side and maybe embodied by having a meshed, latticed or matrix-type structure. This structure allows for a natural bias to a contracted state but enables expansion as needed, dynamically increasing space between the ceiling and the base to accommodate a wide range of finger circumferences and yet permit easy insertion, secure holding and safe removal.

Typically, the base and ceiling are integrally connected together, that is, the receptacle may be a single form piece, made from an efficient production process, such as injection moulding or 3D printing. The receptacle further defines a cutaway or window, where the flexible connecting portion is absent adjacent the distal end of sleeve. This window helps the user define an optimum cutting area, when the receptacle is used alone for blood release and capture (that is despite whether or not a holding device is used). In use the sides of the receptacle cutaway, positioned toward the distal end of the sleeve are configured to visibly frame or part frame an optimal incision area to help better identify this to the user.

In embodiments, the proximal edges or rim of the sleeve are flared outwardly and may have a smooth and/or rounded edge finish about its perimeter that contacts the user and thus provide comfort when the user fits the receptacle fully onto the finger.

The invention herein extends to a finger holding device to enable a useful larger amount of blood to be captured and collected from a finger than devices described in the art.

The invention therefore additionally relates to a finger holding device which comprises a base; an elongate docking structure comprising a docking arm and docking foot; a finger receptacle, optionally in accordance with the above described features disclosed herein, wherein the docking arm is engaged with the finger receptacle and movable there along, such that the device has a closed configuration in which the receptacle is aligned alongside the docking arm and rests adjacent the docking foot and an open configuration in which the receptacle extends from the docking arm and is non-adjacent the docking foot; and an attachment interface for releasably mounting an interchangeable collector attachment to the device.

In embodiments, the finger receptacle may take the innovative form described herein and comprise the features disclosed, or may be provided in a structurally simpler form, unlimited by some or all of said features but which provides sufficient compression of the finger.

The device may further comprise an interchangeable collector attachment, wherein the collector attachment is adapted to temporarily secure one or more fluid collectors to the device for blood collection. The interchangeable attachment component maybe disposable (not reusable) and thus can be removed/replaced when a different form of collector is needed for subsequent use with the finger holding device. This allows the device to be reused with different forms of blood collector on demand; simply attaching the desired type of collector attachment at the attachment interface in the base of the device enables a broad range of different vial types to be used, thereby enhancing the function and capability in blood capture and collection.

Turning to the engagement between the key features: the docking arm is engaged with a finger receptacle, such as the type described herein and movable there along, such that the device has a closed configuration in which the receptacle is aligned alongside the docking arm and rests adjacent the docking foot and an open configuration in which the receptacle extends from the docking arm and is non-adjacent the docking foot.

As disclosed, the finger receptacle part of the device ensures a diversity of finger shapes and sizes can be accommodated in the finger holding device whilst delivering the required technical functionality. The features of the novel finger holder therefore together define a unique opening and closing mechanism, which provides several advantages when the holder is used in home blood capture and collection. However, further advantages are gained from the sliding mechanism, and the structural positioning of cooperating features defining that mechanism when the finger holder and the receptacle are in use.

In embodiments, the arm of the docking structure comprises an internally facing channel with lateral internally facing gripping teeth or equivalent structural means for engaging the receptacle. In embodiments such a structural feature usefully serves to receive a correspondingly-shaped docking engagement feature upon the finger receptacle. The docking engagement feature may be positioned on the topside surface of the receptacle and comprises a rail or raised platform section which is adapted to fit within arm's channel. The gripping teeth of the channel readily secure the edges of this section. These features are adapted by way of their shape and/or dimension to cooperate seamlessly together and enable the arm to move or slide along the length of the receptacle with ease giving rise to open and closed positions of the device.

Once a finger is positioned within the receptacle (in the initial closed/docked position) or beforehand, the user can easily attach or remove a vial to the collector attachment at its collector attachment and interface with their other (or the vial can be place on to the collector attachment prior to positioning on the finger) hand. The holding device is then able to be moved into an open position, where the receptacle and finger are further spaced or distanced from the docking foot. In doing so, two optimal cutting locations, defined by opposing windows of skin, spanning the length on each lateral side, are revealed to the user. The window defined in the receptacle alone helps visibly define the optimum cutting area, blood release and capture. However, use of the receptacle as part of the holding device further aids that identification since the surrounding structure of the holding device visibly provides a clear surround further emphasising the optimum area for cutting.

In embodiments, the receptacle further defines features which enable one or more cutting tools to be used correctly by being guided or physically aligned or secured to the receptacle when used in conjunction with the holder, ensuring its precise location relative to the finger.

The receptacle may comprise one or features which enable a non-integrated cutting tool to be aligned and/or temporarily attached or connected with the receptacle. In embodiments, cutting guides, in particular two or three guide lines, may be present on the outer surface of the support section of the receptacle to provide a visible aid to the user as to where to place the cutting tool (s) to help ensure an incision(s) is made in the relevant position and improved blood release and capture is possible during use. Such features ultimately further help assist with compression while the user is wearing the device and that when a cutting action is made, a consistent depth of cut is achieved.

In embodiments the guides may protrude outwardly from the receptacle thereby providing a physical alignment aid by defining a space which the cutting tool may occupy. The physical guide may comprise at least two fins, creating a receiving corridor into, or through which, the cutting tool may slide or be pushed in order that it is temporarily supported upon the receptacle and in the correct location relative to the skin of the finger for optimal cutting. To cutting tool is better supported since when in the open position the front surface of the cutting tool may rest upon/against the docking foot 106 of the docking station structure of the device.

In further embodiments, where more than one cutting tool is required to be used with the receptacle, three fins spaced around the curved surface of the receptacle may be present. This is to permit two "single use" cutting tools to be mounted to the device simultaneously and multiple cuts to be made in quick succession. Since the time lag needed for changing the tool and re-cutting is avoided and cuts are in slightly different locations, blood capture and collection (of a useful volume) is effective and efficient.

Alternatively, in place of the alignment fins, there may be at least one clamp, or two clamping structures, each of which comprises two opposing members for gripping or latching to the corresponding two opposing sides of the cutting tool, temporarily fitting the cutting tool or tools securely to the device (for better stability) when used therewith.

The user may then apply a cutting action by slicing/cutting with a tool of choice in one, or both, windows of skin. The finger holding device of the invention therefore identifies the optimal areas for cutting to the user whilst retaining flexibility for the user choice of cutting tool.

Cutting in this position and immediately closing the holding device allows the user to hang their arm down in a relaxed position by their side, resting below (lower than) their heart; the user does not see the cuts, or directly witness the flow of blood therefrom and minimal spillage occurs at the wound site.

The general elongate shape of the holding device and position relative to the heart during the flow and collection phase, improves the blood flow from the cutting site. The internal profile, geometry or structures within the device and particularly the base, may additionally promote flow and help guide blood efficiently through the device for swift collection.

In embodiments, an inner compartment of the base defines at least part of an optimised fluidic pathway. The funnelled shape and additional structural features enhance the blood flow further. In embodiments, the base of the device further comprises an aperture connecting the fluidic pathway from the inner compartment to the collector attachment and vial.

The movement between the collector attachment and interface of the base determines a fluidic connection with the inner compartment, in other words the movement enables the fluidic pathway to be opened by unblocking the aperture or blocking it to close the fluidic connection.

In embodiments, the inner compartment of the base may further comprise a fluid directing projection. The projection may extend from the inner surface of the inner compartment of the base, typically, adjacent or proximal to the aperture. In some embodiments, this feature comprises a blood flow director and maybe in the form of a blunt spike.

When an incision is made and the device is closed, the receptacle is prevented from fully re-closing such that the end of a user's finger is enclosed by the inner compartment of the base but is not touching a surface thereof.

In embodiments, therefore the extent of the length of the sliding movement between the receptacle and the holder, i.e. the "open" and "closed" positions as described above, may be pre-determined by additionally useful cooperating features found in embodiments of the receptacle and or the holder.

To this end the receptacle may optionally have a ridge situated centrally on its topside. Preferably, the ridge is situated upon the rail or platform section, herein before described. The ridge is preferably elongated and its total length maybe defined by a cutaway located at each of opposing terminus. The two cutaways act as temporary pit stops in to which a flexible tab of the arm will fall and rest when it reaches either one of the two termini of the ridge (during either the opening or closing sliding movement with the receptacle).

Usefully, when the device is closed, this embodiment facilitates the finger to be held partially within the base of the holder immediately adjacent thereto but without abutting any surface of the inner compartment because the flexible tab rests within the distal pit stop and cannot slide further there along. Additionally, when the device is opened, the flexible tab will rest in the proximal pit stop preventing the arm sliding further and automatically disengaging from the receptacle unless significant further force is applied.

Blood is released from the wound site and flows naturally under gravity downwardly on the lateral sides of the finger, thereby following an optimised internal path and flowing directly from the finger into the base and on to the surface of its inner compartment.

The internal arrangement therefore aids efficient flow of blood through the device to its aperture for collection in the vial/collector fitted with the collector attachment. If present, the blunt spike or projection permits the blood to track a slightly alternative path through to the inner compartment of the device rather than directly via the finger.

Further, where there are two cuts, the projection may permit the two flow paths of blood coalesce at a central point in the device and thus track along the same ultimate path to the aperture. However, in embodiments comprising a receptacle stop, upon closing the device, the finger is held partially within the base immediately adjacent to, without abutting, the surface of the inner compartment. In that embodiment no projection is necessary, since blood flow from the incisions will naturally coalesce at the fingertip end and flow into the inner compartment directly therefrom.

Moreover, with the device comfortable and secure, the user may move around naturally to further increase flow with minimal risk of spillage.

The inventors have found that blood flow is improved well beyond the rates achievable in the prior art devices such as two or three times the rate observed in the use of such devices. Embodiments of the invention achieve rates of blood flow in the range of 0.25mls-0.5mls/per min. Collection is efficient and relatively painless because blood flow is naturally stimulated without direct external force, such as repeated pumping upon near/around the site of cutting, which can cause haemolysis of the red blood cells which is detrimental to the quality of the blood and can confound the testing results. The device is also designed that if required the individual can further increase the blood flow by using a "milking" approach and thus further compressing the device around the finger As a result, limited additional stimulation is required to swiftly collect a sample than is sufficient in volume for multiple testing purposes, such as at least 500 µL or 600 µL but more desirably at least 1ml.

Usefully, the invention allows a patient to independently use the finger holding device to identify an optimal cutting area, release blood, connect a collector and if required remove a vial and thus collect a greater amount of blood with less haemolysis and risk of clotting per cutting action (around 1ml) from their finger than existing self-operated devices and with limited risk of blood leakage, reduced collector time and reduced pain (including a requirement for fewer cuts to achieve the required amount of blood).

The sample volume of around 1ml obtained in the one or more collector chambers/vials, would therefore be suitable for directly sending and processing in lab-based diagnostic tests, without the need to visit a clinician or requiring a trained phlebotomist. The invention and embodiments thereof facilitate a convenient home-based method for blood collection, whereby the user experiences less discomfort or pain than the conventional needle-based method used by the clinician and other devices on the market. Should the user require a greater (or further) sample, the finger holding device can be re-opened and an additional cut in the same window and/or on the other lateral side of the finger can be performed at will and the closure/collection process repeated. The invention therefore extends to use of the finger receptacle and or finger holding device, as described herein, in a method of collecting a blood sample of at least 1ml.

Thus, in a further aspect, there is provided a blood collection assembly kit which comprises the finger holding device as described in any aspect above, together with at least one cutting tool and/or at least one vial for use with the holding device. Each additional component, whether a cutting tool and/or collector chamber (vial), may be sterilised and packaged separately ready for use and/or assembly with the blood collection holding device. Advantageously, the user can effectively utilise the components together for immediate and convenient blood sample collection at home.

Such cutting tools may be provided as part of the kit in preferred embodiments of the invention. Where a kit is provided, cutting tools are selected from those which have a blade undertaking a sweeping or slicing action, for example, following a pendulum-type cutting path. This is opposed to a puncture based mechanism caused by a needle or lancet, as described in methods of the prior art and disclosed in the background here above.

It is recognised by the present authors that use of the cutting tools comprising a blade following an elongate pendulum cutting path will reduce trauma, pain and bruising. In some embodiments the off-the-shelf cutting tool provided with the assembly for use therewith may in embodiments create a cutting path (and thus incision) of up to 2mm depth and/or up to 2-3 mm in length. In such embodiments, a cutting tool provided in the kit may comprise: a casing housing a blade held in a carriage and a guiding track. In operation, the blade and carriage are arranged to slide along the guiding track to define a precise cutting path through which the blade passes in order to make an elongate incision of a precise depth. Such devices are design to provide a clean elongate cut as compared to a puncture hole and thus enhance the benefits provided by the invention.

Off the shelf cutting tool products, such as the sort of tools (referenced for example only) described in US5797940 or US6221089 may be used with the invention.

The cutting tool is operable by the user to make at least one elongate incision in the digit/finger when held in the finger receptacle in the open position of the collection holding device. The kit-style assembly of one aspect of the invention permits the user to undertake cutting at will, meaning a first cut can be made, the holding device closed and blood collected and at a later time, the holding device can be re-opened a further cut can be made. This allows flexibility for the user should there be a location preference for the site of the incision on the digit, and/or to avoid repeat trauma at the same site within a limited time period by altering the incision site. It also allows more blood to be collected from the same digit, without repeated site trauma.

At the distal end of the finger holding device the collector attachment enables connection with at least one collector, such as a chamber, tube or vial, at the interface. The vials may have a push fit mating structure, or similar industry standard to releasably secure a vial to the assembly at the distal interface via a complementary fitting in the collector attachment. Alternatively, a simple thread system into the attachment maybe used until it is temporarily fixed therein. In such embodiments, the vial or collector, vial or collector may have a capacity of 0.4ml to 2.0ml. Each vial can be removed by disconnecting from the collector attachment interface and sealed with a cap or lid, ready for transporting elsewhere (e.g. by post to a lab). In embodiments, the collector attachment and/or the one or more vial or collectors may comprise an agent selected from one or more of an anticoagulant and preservative.

The collector attachment may also include a means, such as a filling control mechanism to selectively block the fluid pathway and temporarily halt the flood of blood into an attached chamber or vial. In embodiments the collector attachment is slidable relative to the base to selectively block the aperture. In other embodiments, the collector attachment is rotatable relative to the base to selectively block the aperture. The attachment interface of the base may comprise retainers to secure the collector attachment and permit that slidable or rotational movement relative to the base to selectively open or close the fluidic pathway by blocking the aperture.

The mechanism for filing control may be more complex. In some embodiments this may comprise a spring ball-valve, optionally connected to an external lever for operating the ball valve and affecting an open or closed status of the fluid path through the collector attachment. Dependent on the capacity of the vial used an empty, sterilised vial can then be attached and if necessary the fluid pathway re-opened and blood collection continued. The mechanism may be activated or halted by operation of a switch or lever which the user can operate manually. Alternatively the mechanism may be automatically halted when a certain volume of blood has been sensed. In some embodiments a sensing mechanism may trigger closure of the aperture to prevent over filling.

In embodiments, the collector attachment is arranged to releasably secure a second vial or collector and define a second fluid pathway between the receptacle and the second vial or collector.

In such embodiments, the filling control mechanism may be operable to open the first fluid pathway and/or second fluid pathway and permit blood flow into the first chamber and/or second chamber. The filling control mechanism may also close the first fluid pathway and/or second fluid pathway and thus stop blood flow into the first chamber and/or second chamber as desired. The filling selection mechanism may be operable in a variety of useful modes, e.g. it may be operated in order to fill the first chamber and second chamber simultaneously, or alternatively it may allow filling in a serial manner.

In such examples, there may be an externally located manual lever on collector attachment to facilitate the desired operation of the filling control mechanism. Alternatively, the filling control mechanism may switch between fluid pathways automatically; closing off the first fluid pathway and opening a second on sensing when predetermined amount of blood has passed through the collector attachment of the finger holding device.

In examples, the invention may be used with one vial or two vials to maximise the amount of whole blood that can be collected. Furthermore, it may be desirable to collect plasma from the blood immediately as there are some analyses methods (for example liquid biopsies) which require plasma samples directly for diagnostic testing. In such embodiments, the vial or collector may have a filter at its proximal end and the plasma may be separated through the filter from the whole blood using gravity or a vacuum from the tube. This can be achieved by collecting two smaller whole blood vials, both of 0.5ml or single larger whole blood vial of 1.0ml. A larger vial may be used to collect up to 2ml whole blood from which more than 500µl plasma may be extracted.

### Brief Description

The invention and various embodiments thereof will now be described with reference to the following figures:
Figure 1 shows a first perspective view of a finger receptacle according to the invention;
Figure 2a shows a first perspective view of a finger holding device, including the finger receptacle, in accordance with a further invention;
Figure 2b shows a perspective view of a further embodiment of the device of the invention shown in Figure 2a, in which the interface structure slidably receives the interchangeable collector attachment during assembly of the device (the receptacle is absent only for simplicity);
Figure 3a shows a perspective view of a fully assembled finger holding device of the invention, wherein two-vials have been attached to collector attachment;
Figure 3b shows a side view of the device in Figure 3a;
Figure 4a shows a cross-sectional view of the finger holding device of the invention in a closed position where the receptacle is docked and the user has inserted a finger. One vial has been attached for use therewith;
Figure 4b shows across-sectional view of the finger holding device of Figure 4a, whereby the device has been pulled to an open position and the receptacle is spatially distanced from the foot of the device permitting access to a lateral window of the user's finger;
Figure 5a shows a side view of the device of the invention in the open position;
Figure 5b shows a perspective view of the device of Figure 5a;
Figure 6a shows a perspective view of the device of Figure 5a/5b during use when a lancet or cutting device can be applied to in an optimised position(s) to enable blood capture from the finger;
Figure 6b shows a perspective view of the device of Figure 6a during use when a lancet or cutting device can be used, in a further, different optimised cutting position(s) on the finger;
Figure 7a and Figure 7b show a perspective views of an embodiment of the device of the invention in which the collector attachment is slidably re-positioned relative to the attachment interface;
Figure 8a and Figure 8b show perspective views of an embodiment of the device of the invention in which the collector attachment is rotationally re-positioned relative to the attachment interface;
Figure 9 shows view of the receptacle comprising alignment lines features in accordance with embodiments of the invention;
Figure 10 shows a view of the receptacle comprising further alignment fin features in accordance with embodiments of the invention;
Figure 11 shows a view of the receptacle comprising further alignment clamping features in accordance with embodiments of the invention; and
Figures 12a and 12b show side views and further 12c shows a perspective view of some of the features involved in determining and controlling positional relationship between the receptacle and the docking arm of the device of the invention.

### Detailed description

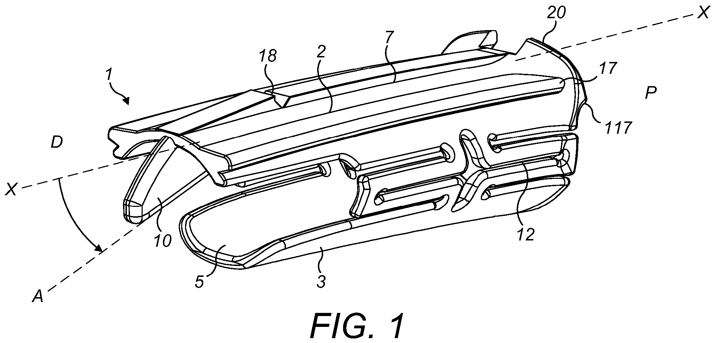

Figure 1 shows a finger receptacle 1 and comprises a generally elongated radially flexible sleeve 2 with a proximal end p and a distal end d. The structure of the sleeve may have a generally tubular structure, including a support base 3 and a ceiling 7. The ceiling and base are flexibly connected to one another, shown here, by side sections 12. In this example, the base, ceiling and side sections therefore also together define an inner surface of the sleeve and which, in cross-section, is approximately circular or oval.

The support base 3 defines at least a part of the inner surface 5 and which base surface is arced or curved, e.g. concave, to comfortably support the rounded underside of a finger, when inserted into the sleeve. Similarly, the ceiling 7 defines at least a further part of the inner surface of the sleeve and is curved to closely cup the topside surface of the finger, further helping to achieve a good fit during use.

Figure 1 shows the receptacle with the sleeve in a resting or contracted state. The overall flexible nature of the sleeve structure 2 permits radial expansion to accommodate the finger but the natural bias of the sleeve is to revert to the contracted state. This arrangement will effect a radial compression on the finger which functions to temporarily secure the receptacle there around and apply an even compression to the whole trunk of the finger. In other words, the sleeve structure will bias radially inwardly (adopting a contracted state) but when urged to expand (radially outwardly) by the presence of a finger having been inserted therein, a radial compression or force is applied evenly by the length of the sleeve, releasably securing the finger within the receptacle. The receptacle sleeve therefore has features that enable a comfortable but, nonetheless, secure fit for various finger sizes.

Here, in Figure 1, the base and ceiling are flexibly connected by the side sections 12 which help boost or enhance the flexible nature of the sleeve. In some cases the material is not necessarily inherently flexible but the structure of the sides of the sleeve will impart this quality to the receptacle as a whole via a clamping compression.

The material of the sleeve may be rigid and be made flexible by the side sections or be inherently elasticated by virtue of the material itself. Typically it may be non-toxic plastic or environmentally friendly for easy disposal. The receptacle may be made from materials such as polypropylene, polycarbonate (PC), polycarbonate acrylonitrile butadiene styrene (PC/ABS), polymethyl methacrylate (PMMA), polystyrene or even metals, such as steel or memory shape alloys. Where an elasticated structure is preferred a strengthened or structured silicone may be selected.

The side portions may have a matrixed, latticed or meshed type of structure. As shown, the flexible side section 12 has a patterned cut-out portion to help facilitate the radial expansion and retraction function of the sleeve. To facilitate insertion and accommodate any finger girth, the general diameter of the sleeve, aided by the flexibility of the side sections, increases. However, the side sections will help resist or limit that increase, to ensure a snug fit of the sleeve about the finger and enhance the blood flow rate at the tip of the finger.

The general elongate nature of the sleeve alone smooths the surface of the finger along its length, providing a more consistent and even pressure which contributes to technical effect achieved by the compression actions, described herein.

As shown, the ceiling 7 of the sleeve defines longitudinal axis X. The receptacle further includes at least one angled, distal projection 10. The projection extends away from the longitudinal axis of the ceiling, being angled inwardly (see arrow) relative to a central axis (not shown) of the receptacle. As shown in Figure 1, the angled projection maybe shaped as a triangular wedge, extending a short way beyond the distal end of the ceiling. The projection 10 extends downwardly having limited angle A of approximately between 10-40 degrees, preferably 15-25 degrees, relative to the longitudinal axis X of the ceiling. This projection further assists with the technical functionality of the receptacle and is particularly helpful when the receptacle is used with or as part of a device or assembly for blood capture. The angled projection is adapted such that when a finger is fully inserted in to the sleeve when it reaches the distal end of the ceiling the projection contacts a nail of the finger. The user may therefore continue to push the sleeve over the finger, whereby the projection pushes downwardly at angle A on to the nail, the fingertip is therefore subjected to a second compression action or force and over hangs the support base. The user therefore finds that the two compressive actions combined help the receptacle to remain securely fitted, even when the user changes position, for example, lets his or her hand relax by one's side and provide complimentary compression to the finger to optimise blood flow when the receptacle is used with a device for blood capture.

At the proximal end P of the structure sleeve, located at proximal end of ceiling 7, and/or the support base 5, there may be a flanged or lipped section 20, present to ensure comfort such that no sharp or harsh edge is pressed against the skin webbing between fingers on the hand when the finger is inserted into the sleeve of the receptacle.

The receptacle may have further advantageous features designed to optimise existing function and enhance use in specific applications. For example, although not essential in a primary form, the topside of the sleeve ceiling structure 7 may include specific features which enable a secure, movable interconnection with a finger holding device, which is a further aspect of the invention disclosed herein.

Such a finger holding device may be provided, as described below, for use in blood capture.

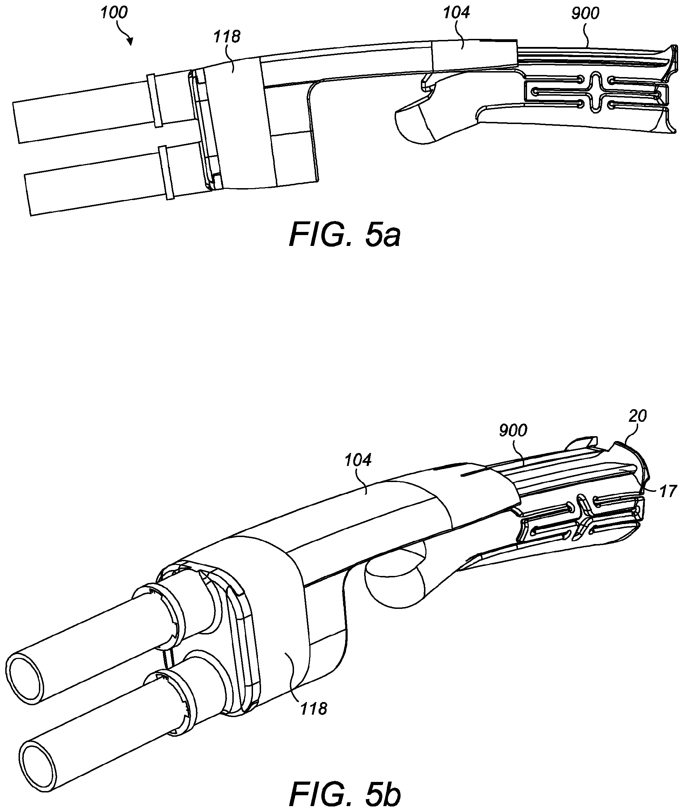

For example, when used as part of the finger holding device embodiments of the receptacle may have further useful technical features. In some embodiments there may be an elongated linking or connecting structure (s) such as a mono-rail or, as shown clearly in Figure 1 and later in Figure 5b, rails 17 which are positioned along the topside surface of ceiling 7 of the sleeve of the receptacle 1.

This rails feature allows secure engagement of receptacle 1 with (an arm 104) of the finger holding device 100. Although the feature does not have to embody rails specifically, it must act to facilitate engagement or link the finger receptacle with the device and permit a limited lateral movement between the device and the receptacle. In such embodiments, the lipped section 20 of the receptacle 1, located at a proximal end of the top surface of the sleeve ceiling 7 may also partly act to prevent the sleeve of the receptacle going too far into the finger holding device as well as providing user comfort during finger insertion and fitting, as mentioned above in relation to Figure 1.

As will be described below, when the finger is inserted into the device and the sleeve is closed with the docking arm, the lipped/flared section 20 outer edge will naturally rest against the corresponding outer edge of the arm 104, the latter edge being shaped to receive the former. In later described embodiments, after fully engaging the sleeve and docking therewith a flexible tab of the docking arm 102 may naturally rest in a cutaway formed in the end of the flared section to temporarily secure the two together.

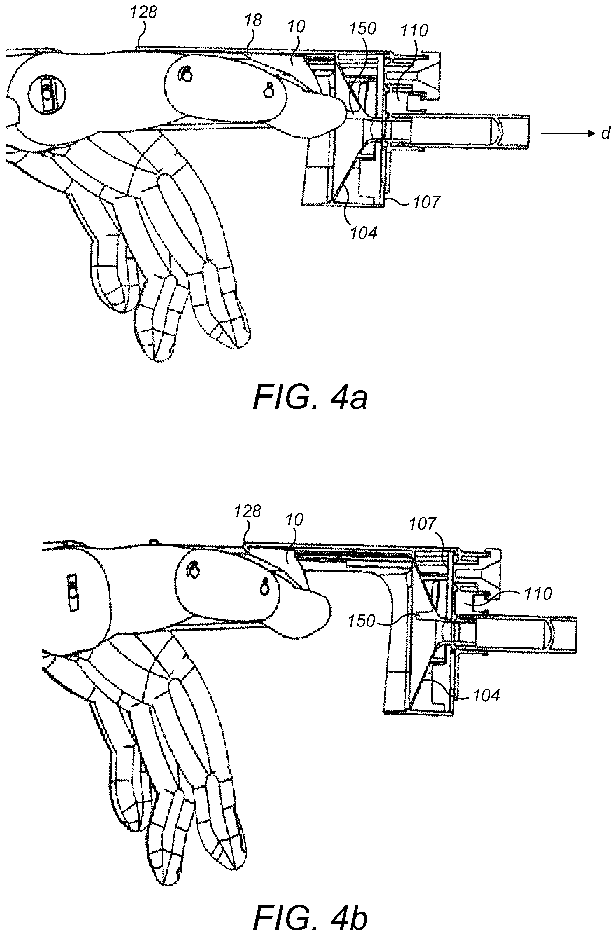

Other features may be present to help prevent automatic disengagement of the finger receptacle from device when it is pulled by the user and the device is opened. For example, a recessed stop 18 may be present in a central location along the topside of the receptacle 1 between the rails 17. This will be described further in reference to Figures 4a and 4b.

The receptacle 1 and device 100 are operable together to provide many advantages as will be discussed in more detail in relation to the device of the invention further below.

In examples, the receptacle 1 is formed of one single moulded piece, that is, the features of the structure described here above are inherently integrated during manufacture by plastic injection moulding or 3D printing. However, it is conceivable that some of these features may not be entirely integral (e.g. made by single moulding process) but are nonetheless able to be present in the device.

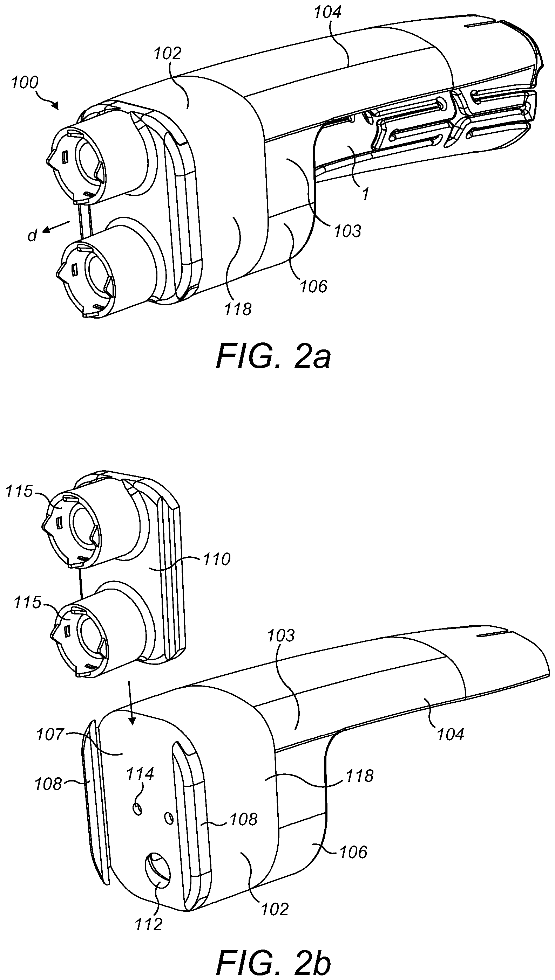

Figure 2a shows a finger holding device 100 according to the invention, comprising a docking base 102 and an elongate docking structure 103. In examples, the base 102 and docking structure 103 are inherently moulded or made as one, their borders with one another only being independently referenced to help illustrate structural positioning relative to other features of the device more clearly. Elongate docking structure 103 and base 102 are shaped and dimensioned to surround a finger receptacle 1, of the type disclosed above.

The finger receptacle 1 of the device 100 is received within docking structure 103 and held thereon by an elongated arm 104 which extends from a docking foot 106, which structurally borders and sits adjacent the base 102.

The receptacle 1 is movably connected to the finger holding device 100 via a recessed portion or channel provided in the underside of the elongate arm 104. As discussed, the receptacle comprises rails 17, which sit within the channel of the arm 104, connecting the receptacle 1 for slidable movement therewith.

In the closed position; the configuration shown in Figure 2a, the finger receptacle 1 is received, half-framed within the L-shape of the arm 104 and foot 106 of docking structure 103, the receptacle 1 being docked.

It is noted that the foot 106 and part of the arm 104 of the docking structure 103 adjoin docking base 102 to define an inner compartment 104.

As can be seen clearly with reference to Figures 4a and 4b, the internal cross-section of inner compartment 104 of docking base 102 and foot 103 is generally funnel or bath-shaped and tapers with rounded sides to define an inner fluid pathway. The pathway leads to an aperture located in the attachment interfacing surface 107 of the base 102.

Referring back to Figure 2a and 2b, the attachment interface 107 of the device can be viewed clearly. The attachment interface or interfacing surface is associated with, or inherently part of, the docking base 102. The attachment interface 107 and may be an integral part of the structure of the docking base 102, but in either case, this feature enables a releasable connection with an interchangeable collector attachment 110 and thus acts as an interface for attachments mounted at the distal end of the device.

As shown in figure 2b, the precise embodiment of collector attachment 110 utilised with the device 100 may differ and thus advantageously can be interchangeable to make the appropriate selection of collector; at least one attachment is always provided.

In the example shown, docking base 102 comprises attachment interface or interfacing surface 107 and interface structures: side elements 108. As provided here, the side structures at the interfacing surface may incorporate means to secure the edges of a collector attachment to the device, allowing for different types of collector attachment to be slidably interchanged therewith.

In Figure 2b, a generic two-vial connector attachment 110 with typical mating connectors 115, as shown, would be suitable for connection with standard vial(s) having a corresponding mating structure.

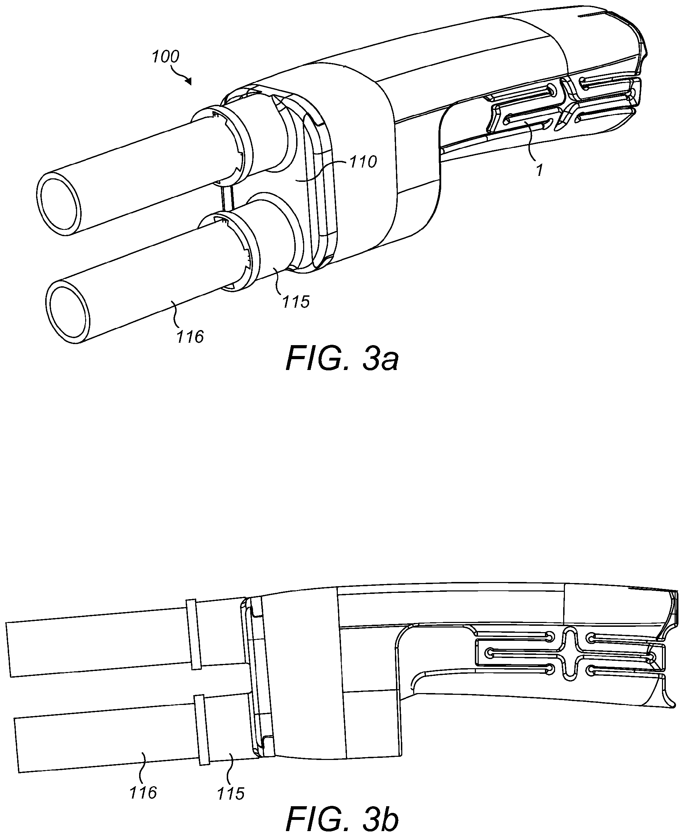

Figures 3a and 3b also show perspective and side views of the device 100 with examples of standard vials 116, e.g. micro-containers, fitted into place at the standard connectors 115, such as push fit, screw-fit, luer-lock or similar, provided in collector attachment 110.

In the field of blood capture having a modifiable device would be very useful, especially where this feature is present at the interface of the base, i.e. as part of the collector attachment. Thus, in alternative embodiments, the collector attachment 110 shown in Figure 2b could be slidably replaced at the interface with a different, specific collector attachment, to permit non-universal connection structures on blood collector vials, or specialist units such as plasma filtration units to be used with the device, further enhancing device capability and functionality of the finger hold device.

Referring again to the example shown in Figure 2b, the surface interface 107 of docking base 102 or its surface may comprise collector attachment retainers 114, embodied as small recesses or apertures for temporarily receiving corresponding features, such a dimples or projections, which are present on the attachment structure 110. In versions of the device where interchangeable attachment collectors 110 are desired, these features click together to prevent accidental slipping or sliding of the attachment structure, relative to the surface of interface 107 (for example, when the blood collectors or vials are fitted using light force).

In embodiments, especially where it is desirable to use single form processes to manufacture the device, the collector attachment 110 maybe inherently secured, or as mentioned above be a part of the physical surface interface 107 or otherwise adjoined with the docking base in a permanent manner.

The attachment interface 107 and associated structures may also allow the device to be adapted such that in some embodiments the mechanism of use with collectors, such as vials provides further technical advantages or functionality during blood capture. These examples, such as those enabling rotational collection mechanisms, will be described in further detail below.

Referring back to Figure 2a and now to Figure 4a, the sliding connection between the receptacle 1 and the docking structure 103 means that the device is operable to permit movement in the distal direction d of the docking base 102 and 106 foot away from the receptacle and so the device adopts a different, open, configuration to enhance blood capture. See particularly Figures 4a and 4b where the device is shown is closed and open positions, respectively.

Once a finger is present within the sleeve of the receptacle 1, as shown in Figure 4a particularly, the user can grip the device, for example, using flat side surfaces 118 (see Figure 2a) of docking base 102 and pull (in a distal direction d) relative to the user's finger to open the device. As the user pulls in direction d, the underside recessed portion in the top surface of arm 104, being slidably connected to rails 17, located on the topside of the finger receptacle 1 permits the docking structure to move, as a whole, along the rails of the finger sleeve until its catch 128 reaches and drops to rest in recessed stop 18. The catch and recess together prevents further sliding movement in direction d and an automatic disengagement of the arm 104 from receptacle 1 (unless further overriding force is used).

However, this distal movement, as indicated in Figures 2a, 2b, 4a and 4b, creates a clear space between the foot 106 of the docking structure 103 and the receptacle 1. That is the receptacle extends from the docking arm and is no longer adjacent the docking foot being spatially distanced from the docking foot and base and in a closed configuration in which the receptacle is docked alongside the elongate docking structure arm and is immediately adjacent the docking foot.

The holding device in the open position creates two optimal cutting locations or visible windows of skin on lateral sides of the finger. Furthermore, the user having his or her finger secured in the receptacle experiences a combination of compression actions which optimise blood flow for subsequent capture. The radial compression of the sleeve continues to clamp, squeezing circumferentially around the body of the finger, increasing blood flow towards the tip of the finger. The flow for improved release rate is further enhanced when the receptacle is massaged externally by the other hand to further direct the blood flow to the distal part of the finger as well causing increased inward flow into the finger. Further, the projection focussing pressure downwardly in a second action enhances the localised blood flow at the tip of the finger towards the cutting zones and causes these areas to be locally engorged with blood.

The combination of these compressive actions permits the device including its receptacle increasing the blood flow rate and the amount released when a cutting action is applied to one or both windows of skin revealed by the device in the open position shown in Figure 4b. The user ensures that at least a first collector is suitably connected at the interface attachment and has been positioned therewith such that the aperture 112 is open and a first fluid pathway is formed, thereby connecting the internal compartment 104 of the base of the device (where the blood will initially flow) to the collector, such as a microtainer vial.

The finger holding device presents the optimal areas for cutting (and which may include markers on the device to assist the user in applying the cutting device in the optimal location(s)) to the user whilst retaining flexibility for the user choice of cutting tool). The user may then apply a cutting action by slicing/cutting with a tool of choice in one, or both, visible windows of skin.

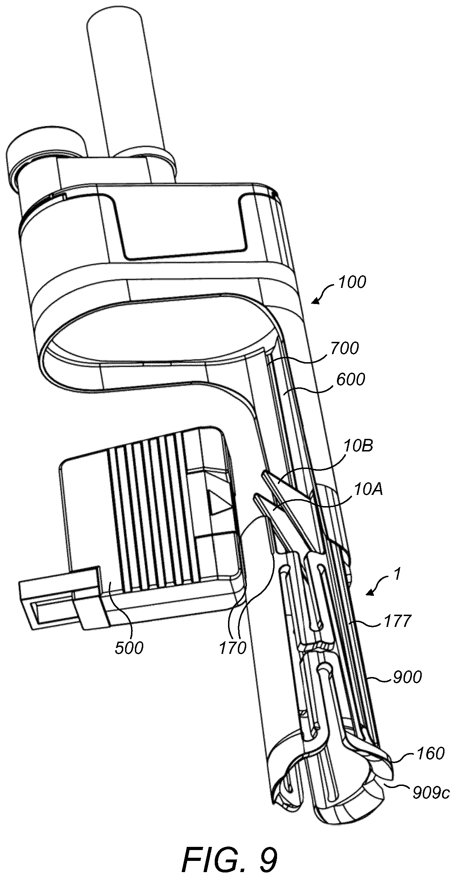

In some embodiments, the support or base section 3 of the receptacle 1 comprises one or more features which enable the cutting tool to be aligned and/or temporarily attached or connected with the receptacle. Cutting lines or guides 170, in particular two or three guide lines, may be present on the outer surface of the support section of the receptacle to provide a visible aid to the user as to where to place the cutting tool (s). This helps ensure an incision(s) is made in the relevant position and improved blood release and capture is possible during use of the current invention. The example form of such guides is shown in Figure 9.

Figure 9 additionally shows how the proximal rim 160 of the sleeve 1 maybe flared outwardly and further may have a smooth and/or rounded edge finish about the edge perimeter that contacts the user and thus provides comfort during use, when the user fits the receptacle fully onto the finger.

Figure 9 also exemplify a receptacle having two spaced distal projections; 10A and 10B. The projections angle inwardly relative to a central axis X of the receptacle. The angled projections are shown here as triangular wedges, extending beyond the distal end at an angle approximately between 10-40 degrees, preferably 15-25 degrees, relative to the longitudinal axis X of the ceiling. This set of projections further assists with the technical functionality of the receptacle and this feature is particularly helpful when the receptacle is used for blood capture. The angled projections are adapted such that when a finger is fully inserted in to the sleeve and reaches the distal end of the ceiling the two projections contact a nail of the finger and apply an even pressure thereacross and downwardly on to the nail at angle A.

In some embodiments, to improve co-operation between the receptacle and the cutting tool and further facilitate ease of use, the guides may protrude outwardly from the receptacle thereby providing a physical alignment aid by defining a space which the cutting tool may occupy.

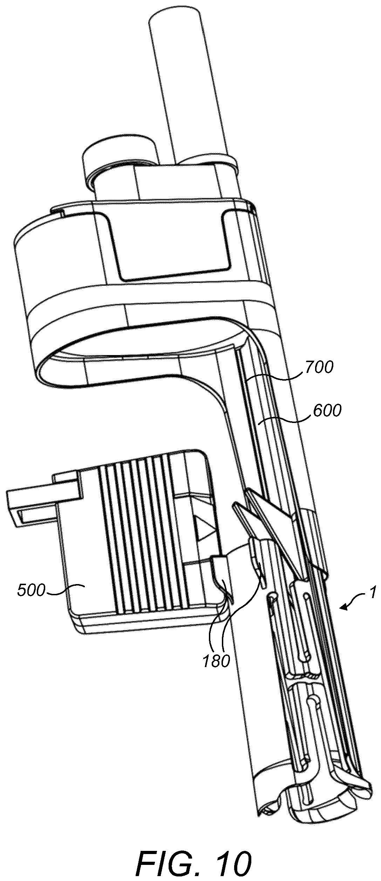

As shown in Figure 10, the physical guide may comprise at least two fins, creating a receiving corridor into, or through which, the cutting tool may slide or be pushed in order that it is temporarily supported upon the receptacle and in the correct location relative to the skin of the finger, for optimal cutting. The cutting tool is better supported since when it is in the open position, the front surface of the cutting tool may rest upon/against the docking foot 106 of the docking station structure of the device.

In further embodiments where, for example, more than one cutting tool is required to be used with the receptacle, three fins 180 are present and spaced around the curved surface of the receptacle. This example permits two "single use" cutting tools to be mounted to the device simultaneously and multiple cuts to be made in quick succession. Since the time lag needed for changing the tool and re-cutting is avoided and the cuts are able to be made in accurate but slightly different locations, blood capture and collection (of a useful volume) is effective and efficient.

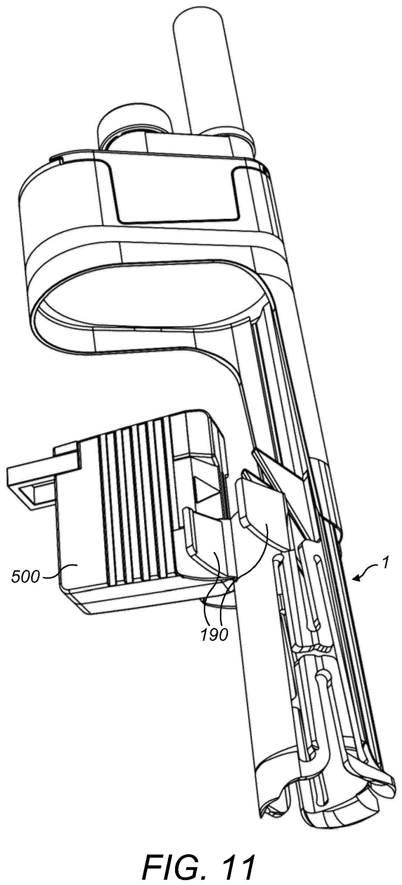

Alternatively, in place of the alignment fins, there may be at least one clamp, or in examples shown in Figure 11, clamp structures 190, each of which comprises two opposing members for gripping or latching to the corresponding two opposing sides of the cutting tool 500, temporarily fitting the cutting tool or tools securely to the device (for better stability) when used therewith.

After cutting in this position, the user subsequently removes the cutting tool and immediately closes the finger holding device by pushing on the docking structure 103 and/or gripping the device using flat side surfaces 118 and pushing in the opposite direction to d, bringing the base 102/foot 106 back toward the receptacle 1. As the arm 104 slides returning along the rails 17 the device adopts the closed configuration again.

Usefully, when the device is closed, the finger is able to be held partially within the base of the holder and immediately adjacent thereto but without abutting any surface of the inner compartment. Figures 9, 10, 11, 12a, 12b and 12c highlight some of the features of the embodiments that enable further advantage:
As shown in Figure 9 the arm of the docking structure comprises an internally facing channel 600 with lateral inwardly facing teeth 700 which engage the receptacle 1 with a light grip. A rail 17/177, positioned on the topside surface of the receptacle 1 and fitting within channel 600, is secured at its edges by the inward grip of the two opposing teeth 700.

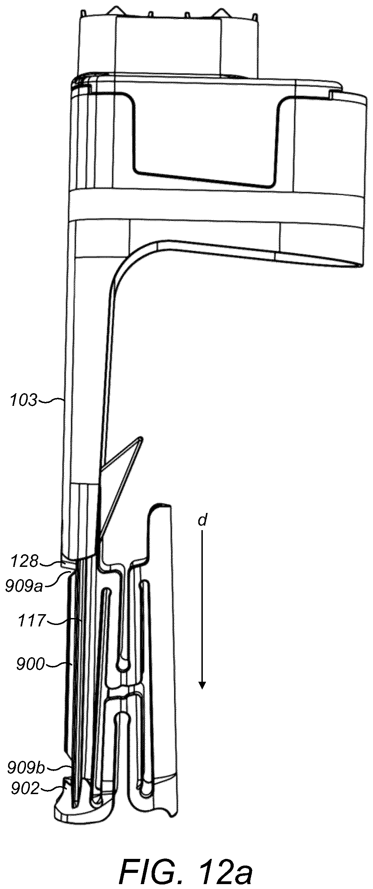

Consequently, the arm can slidably move along the length of the rail of the receptacle with ease, giving rise to the open, closed and in some cases a partially closed positions of the device. The distance of the sliding movement and position of the arm relative to the receptacle is enabled or controlled by further features in various embodiments of the device, as shown and described herein:
In these examples, shown particularly in Figures 9 and 12a, the receptacle has an elongated ridge 900 situated centrally on the rail but with a shortened length relative thereto, the length being defined by a cutaway 909a, 909b located at each end. The two cutaways 909 are recessed relative to the ridge 900 sufficiently to retain catch 128 temporarily. In other words, as latter shown in the positional Figures of 12a and 12b, when catch 128 reaches either one of the two termini at the end of the ridge 900 (during sliding movement in order to open, close or partially close the device) the flexible catch 128, will drop into the cutaway section 909a or 909b of the receptacle's topside surface and acting as a spring tab will lightly rest there until external force, made by the user, causes it to dislodge it from its recessed position within 909a or 909b to rise onto the ridge 900 again.

When the device is fully open as shown in Figure 12a, the catch 128 rests in the distal cutaway 909a preventing the arm slidingly disconnecting from the receptacle completely. The catch 128 and cutaways 909a of the device together prevent further sliding movement in direction d and thus automatic disengagement of the arm 104 of the elongate holder 103 from receptacle 1 (unless further larger overriding force is used).

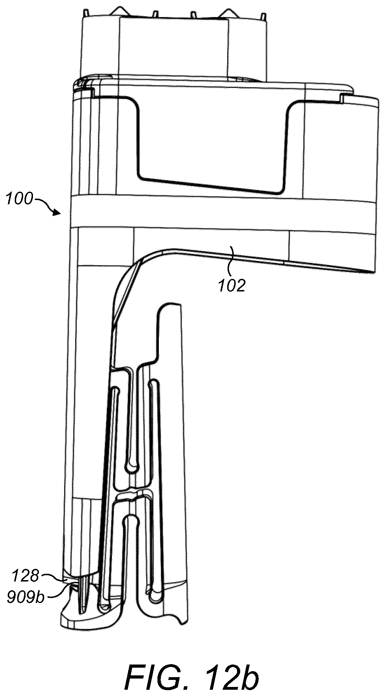

Whereas, as shown in Figure 12b, when the device is moved towards the closed position, the flexible catch 128 may rest within the cutaway 909b. Here, the docking arm is interrupted from sliding further along the rail without additional force. In this embodiment, the finger is optimally held within the base 102 of the holder for blood collection but without the device or finger inserted therein contacting any surface of the inner compartment 104.

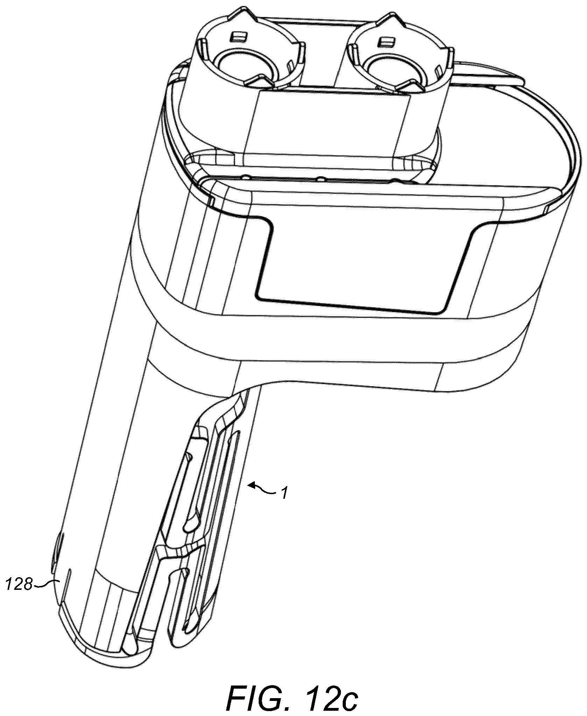

Additionally, some embodiments of the device may include a further cutaway 909c, formed in the end of the flared section of the sleeve of the receptacle 1. This cutaway permits the device to be temporarily secure closed position, in which the docking arm 102, has fully engaged/engulfed the sleeve of the receptacle. In this example, use of a further external overriding force by the user in the opposing direction to d will cause the catch 128 of the docking arm 103 to move out of the cutaway 909b and briefly encounter an additional short ridge 902 on the receptacle (not shown but see Figure 12a) which its rises over before resting again in cutaway 909c (not shown here but see Figure 9) to fully close the device, as shown in Figure 12c.

Turning back to the use, once the incisions have been made in the skin windowed sections and the device re-closed, the user lets their arm hang down by their side in a relaxed position, resting below (lower than) their heart and blood capture and collection will initiate. The user can as required move around without risk of spillage and complete other tasks which may further increase the blood flow. The finger holding device and its correct use thereof permits that the user will not need to see the cuts or flow of blood therefrom once cutting is complete. The elongate shape of the holding device and position relative to the heart during the flow and collection phase, further improve the blood flow from the cutting site(s).

The internal profile or geometry of the device additionally promotes or helps guide blood efficiently during capture and collection. As shown in Figures 4a and 4b, a further internal feature located internally within the base 102 of the device helps efficiently direct the flow of blood into the inner compartment of the base which defines an inner funnel or bath. In some examples, this feature comprises a finger blood flow director 150 and maybe in the form of a blunt spike. Here, the blunt spike extends outwardly as a longitudinal projection from the internal wall of the inner compartment 104 and is located proximal or adjacent aperture 112. Once the device is closed position, the pad or finger tip of the user is lightly pushed inwardly as it rests on to the blunt surface of the spike.

The blunt spike has the further advantage that is helps finger alignment within the device, although additional indicators may be present on the device to assist with this function. Indicators may be in the form of a label, ink, embossing, etching or aperture which permits the user to visually confirm that that the finger is positioned correctly in the receptacle and device.

Blood flow rate and collection rate is able to be optimised by the combination of several factors due to features of the device and receptacle. The multiple compression actions of the receptacle, the possibility for a resting placement in the device permitting gravity to naturally enhance flow and features that cause blood flow to follow an efficient pathway through the device for collection.

Once the cut(s) is made the blood flows readily from the lateral side(s) of the finger toward the fingertip. In the examples shown in Figure 4a and 4b, the blood meets the spike 150 and is directed down its length towards the bowl of the inner compartment 104 in the base, efficiently funnelling the blood into the aperture. The external walls the base 102 and foot 106 help contain blood spill from the wound site(s) by partially/fully overlapping the cutting sites once the device is in the closed position.

With the device secured the user may move around naturally to further increase flow for optimal collection. Light further compression may be applied around the receptacle to "milk" the finger but it is not essential in order to collect a sample of at least 500 µL or 600 µL and most desirably at least 1ml.

Direct external force, such as repeated pumping upon and around the site of cutting to extract suitable quantities of blood is not required. Physical force may cause haemolysis of red blood cells and can be detrimental to the quality of the blood. Very limited manual stimulation is required, if any. The invention is advantageous since it minimises, or even avoids, the need for techniques which may impact quality and thus reduces chance that the sample obtained will be unsuitable for analytical/diagnostic testing.

The invention allows a patient to independently use the finger holding device to identify an optimal cutting area, release blood, connect a collector and if required remove a vial and thus collect a greater amount of blood with less haemolysis and risk of clotting per cutting action (around 1ml) from their finger than existing self-operated devices and with limited risk of blood leakage, reduced collector time and reduced pain (including a requirement for fewer cuts to achieve the required amount of blood).

The sample volume of around 1ml obtained in the one or more collector chambers/vials, would therefore be suitable for directly sending and processing in lab-based diagnostic tests, without the need to visit a clinician or requiring a trained phlebotomist. The invention and embodiments thereof facilitate a convenient home-based method for blood collection, whereby the user experiences less discomfort or pain than the conventional needle-based method used by the clinician and other devices on the market.

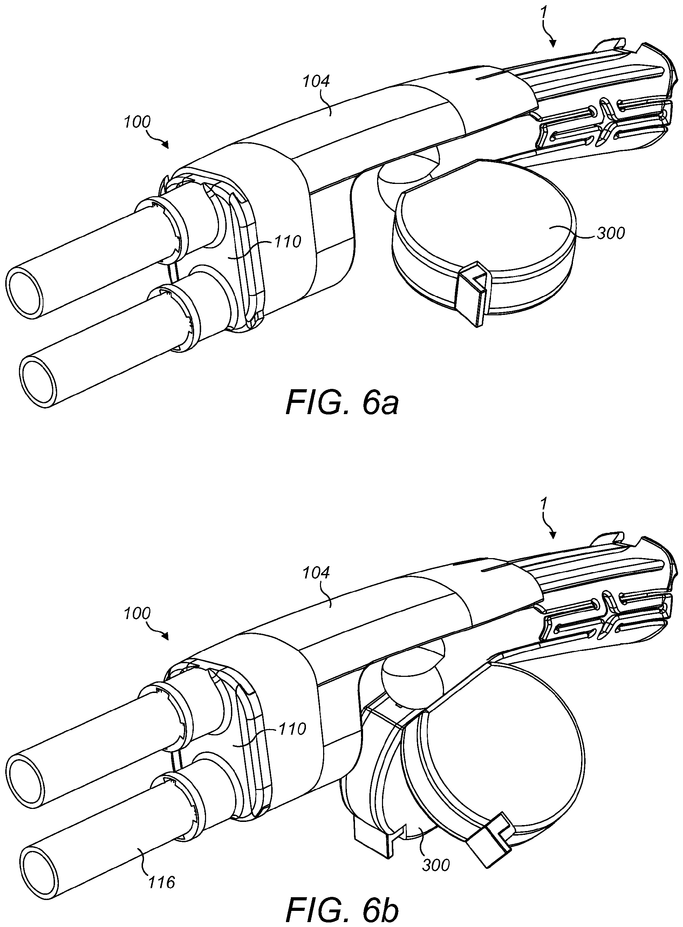

Should the user require a greater (or further) sample, the finger holding device can be re-opened and an additional cut in the same window and/or on the other lateral side of the finger can be performed at will and the closure/collection process repeated. For example, as shown in Figures 6a and 6b, a first cut, followed by a second cut can be made to increase the amount of blood captured and collected using the device. Use of the finger receptacle and/or finger holding device, as described herein is therefore pertinent in a method of collecting larger blood samples, such as those of at least 1ml.

Thus, in a further aspect, there is provided a blood collection assembly kit which comprises the finger holding device as described, together with at least one cutting tool and/or at least one blood collection vial for use with the holding device. Additional components, whether a cutting tool and/or collector/vial, may be sterilised and packaged separately ready for use with the finger holding device. Advantageously, the user can effectively utilise the components together for immediate and convenient blood capture and collection at home.

Off the shelf cutting tool products such as the sort of tools described herein may be used with the invention. An example of such a tool is shown in Figure 6a. Such cutting tools 300 may be provided as part of the kit. In some embodiments the off-the-shelf cutting tool provided with the assembly for use therewith may comprise a blade and mechanism to create an elongate cutting path, in embodiments the path is up to 2mm depth by 2-3 mm length. In such embodiments, a cutting tool provided in the kit may comprise: a casing housing a blade held in a carriage and a guiding track. In operation, the blade and carriage are arranged to slide along the guiding track to define a precise cutting path through which the blade passes in order to make an elongate incision of a precise depth. Such devices are design to provide a clean elongate cut as compared to a puncture hole or prick test.

Such cutting tools are typically operable by the user to make at least one elongate incision in the digit/finger when held in the finger receptacle in the open position of the finger holding device as shown in the positions in Figure 6b. The kit-style assembly of one aspect of the invention permits the user to undertake cutting at will, meaning a first cut can be made, the holding device closed and blood collected. At a later time the holding device can be re-opened a further cut can be made. Alternatively, immediately thereafter the first cut, a second cut can be made before the device is closed. This allows flexibility for the user should there be a location preference for the site of the incision on the digit within the window of skin visibly presented by the receptacle and device. This allows the user to avoid repeat trauma within a limited time period to the same incision site whilst still cutting in an optimised position on the finger. As illustrated in Figure 6b, the device facilitates a larger sample, than typically possible from such devices, to be released from an optimum position on the finger and thereafter collected in vial 116 from the same finger (and without unnecessary repeat trauma).

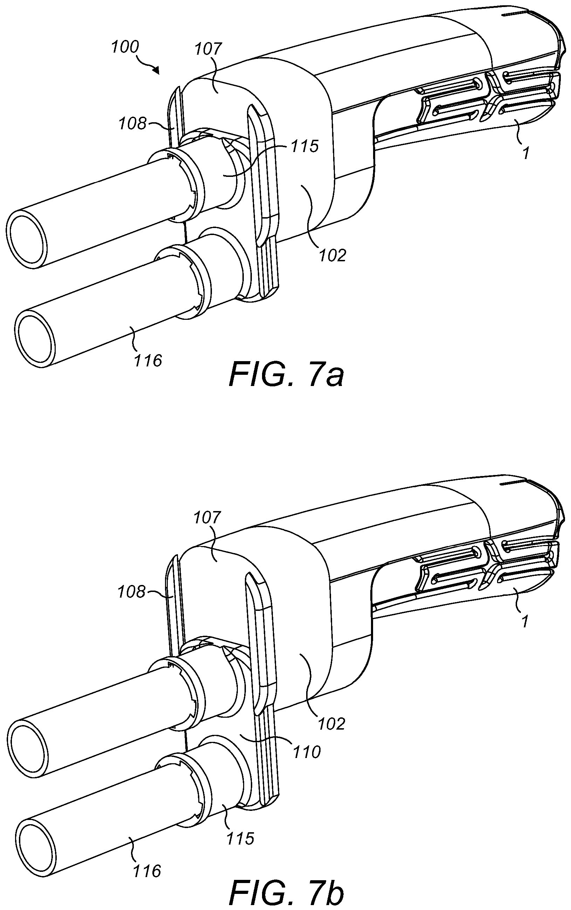

As shown in Figure 7a and Figure 7b, at the distal interface 107 of the base 102 of the finger holding device 100, the collector attachment 110 enables a slidable connection with at least one collector, such as a chamber, tube or vial 116. The vials may have a push fit mating structure, or similar industry standard to releasably secure a vial to the assembly at the distal interface attachment via a complementary fitting 115 in the collector attachment 110. A simple thread connection between the vial and fitting of the collector attachment maybe used to temporarily affix the vial to the device 100.

A sliding movement between the collector attachment 110 and the base 102 at the interfacing surface 107 of the device allows a user to selectively open a first fluidic pathway between the funnelled compartment of the base 104 and the microtainer vial. Figures 7a and 7b show the collector attachment 110 affixed to the vials 116 and adopting different positions by a slidable movement relative to the interface surface 107. When an incision is made and the device is closed, blood flows through the internal path of the device, as described previously. If the collector attachment is positioned such that the aperture 112 in surface 107 is aligned with the vial, blood will continue to flow from the inner compartment of the base of the device into the vial 116. The collector attachment may have positioning visual markers and/or tactile feedback indicators, so the user can easily see/feel whether the first fluid pathway is connected and the aperture is open, or the aperture is closed.

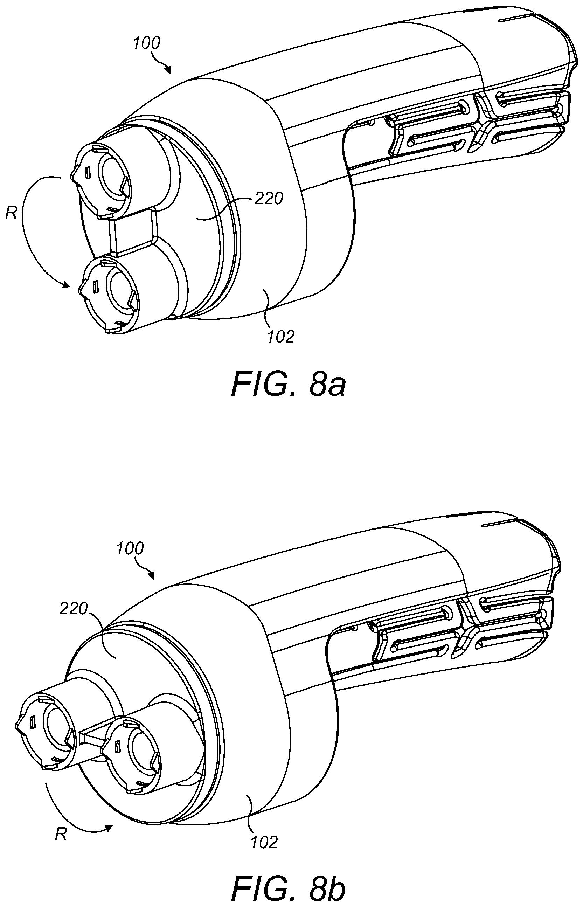

Alternatively, the collector attachment 220 may interface with the base 102 such that a rotational movement is undertaken with respect to the base. An example of this embodiment is shown in Figures 8a and 8b, where the collector attachment 220 comprises a turntable mechanism and is rotatable in direction R with respect to the surface interface 107 (not visible). As with the embodiment described above (shown in Figure 7a and 7b) movement by the collector attachment 110, 220 is utilised position the vial in a location which permits blood collection, when desired. The collector attachment 220 is twisted with respect to the base 102 to block or unblock the aperture within surface interface. The fluidic pathway from the inner compartment is therefore opened to allow blood to run into the vial (not shown here) or prevented to halt collection.

In the examples shown, the collectors or vials used may have a capacity of 0.5ml to 2.0ml. Such vials normally have visible fill lines so the user can see when the required collector volume is reached during use. Such tubes tend to be of approximate dimension 13 x 75 mm but are not limited to this and hold a volume of 250-500µL or 400-600µL. After use, when the tube is unscrewed from the assembly, the cap is replaced, sealing the tube containing the blood (or plasma) for safe transport. In simple embodiments, the assembly arrangement may comprise a collector attachment with only a single vial connection capability and thus may not include any complex functions associated with multiple vial attachment.

In some embodiments the collector attachment/base and interface 110/107/102 may include a means for managing air blockages to allow any trapped air to escape from the fluid pathway of the device effectively without compromising blood flow to the vials. Such means may include an escape flue, for example.

Where the device of the invention is used as part of a packaged kit, after use with the cutting and collector components, the filled collector vial or tube can be removed by disconnecting from the collector attachment and sealed with its cap or lid, ready for transporting elsewhere. In embodiments, either the collector attachment and/or the one or more collectors may comprise an agent selected from one or more of an anticoagulant and preservative. The capped blood vials/tubes are then ready to by transported e.g. by post to a lab without needed a clinician's involvement, or in some locations immediately processed.

The rest of the disposable assembly can then be replaced into the package, optionally re-sealed and discarded safely.

## Claims

1. A finger holding device comprising:
a base;
an elongate docking structure comprising a docking arm and docking foot;
a finger receptacle, for finger compression, wherein the docking arm is engaged with the finger receptacle and movable there along, such that the device has a closed configuration in which the receptacle is aligned alongside the docking arm and rests adjacent the docking foot and an open configuration in which the receptacle extends from the docking arm and is non-adjacent the docking foot; and
an attachment interface associated with the base for releasably mounting an interchangeable collector attachment to the device.

2. The finger holding device according to claim 1, wherein the finger receptacle further comprises an elongated, flexible sleeve having a length defined between a proximal end and a distal end, the sleeve further comprising a support base, a ceiling and a top surface having a longitudinal axis X, whereby the sleeve is adapted to bias toward a radially contracted state and when urged into a radially expanded state effects a radial compression action about the length to releasably secure a finger within the receptacle; and at least one angled projection extending from the ceiling and top surface at the distal end of the sleeve, the at least one angled projection angled inwardly from the longitudinal axis X of the ceiling and adapted to contact a nail of the finger releasably secured in the sleeve at the distal end of the receptacle to effect a second compression action.

3. The finger holding device of claim 1 or 2, wherein the device further comprises an interchangeable collector attachment, wherein the collector attachment is adapted to temporarily secure one or more fluid collectors to the device.

4. The finger holding device according to any previous claim, wherein the docking arm is engaged with a top surface engagement structure of the finger receptacle and is slidable there along to facilitate movement of the device between the open configuration and the closed configuration, or to a partially closed configuration.

5. The finger holding device according to claim 4, wherein the docking arm comprises a channel for receiving the top surface engagement structure of the finger receptacle.

6. The finger holding device according to any previous claim, wherein the docking arm further comprises a flexible catch for temporarily engaging the receptacle and maintaining the open configuration, the closed configuration or a partially closed configuration.

7. The finger holding device according to any previous claim, wherein the base defines an inner compartment partially defining a fluidic pathway within the device.

8. The finger holding device according to claim 7, wherein the base further comprises an aperture connecting the fluidic pathway from the inner compartment to the collector attachment.

9. The finger holding device according to claim 8, wherein the collector attachment is slidable or rotatable relative to the base to selectively block the aperture.

10. The finger holding device according to claim 9, wherein the attachment interface of the base comprises retainers to secure the collector attachment and permit the slidable or rotational movement relative to the base.

11. The finger holding device according to any previous claim, wherein the collector attachment is adapted to secure one or more collectors.

12. The finger holding device according to claim 11, for use with at least one cutting tool in a blood capture kit.

13. A blood capture kit comprising the finger holding device of any of claims 1 to 11 and at least one cutting tool.

14. The blood capture kit according to claim 13, for assembly and use with one or more fluid collectors, in a blood collection assembly kit.

15. A blood collection assembly kit according to claim 13 or 14, further comprising one or more fluid collectors for assembly therewith and wherein the one or more collectors has a capacity of 0.5ml to 2.0ml.
